# EUROPEAN PATENT SPECIFICATION

(11) **EP 4 450 556 B1**
(45) Date of publication and mention of the grant of the patent: **28.01.2026**
(21) Application number: 24161560.8
(22) Date of filing: 05.03.2024
(51) Int. Cl.: C08L 23/28, C08K 3/22, A61M 5/00, A61M 5/315

(54) **MEDICAL RUBBER COMPOSITION AND MEDICAL RUBBER PRODUCT**
MEDIZINISCHE KAUTSCHUKZUSAMMENSETZUNG UND MEDIZINISCHES KAUTSCHUKPRODUKT
COMPOSITION DE CAOUTCHOUC MÉDICAL ET PRODUIT DE CAOUTCHOUC MÉDICAL

(30) Priority: 21.04.2023 JP 2023069790
(43) Date of publication of application: 23.10.2024
(73) Proprietor: Sumitomo Rubber Industries, Ltd., Kobe-shi, Hyogo 651-0072 (JP)
(72) Inventor: MORITA, Shumpei, Kobe-shi, 651-0072 (JP); MATSUTANI, Yuichiro, Kobe-shi, 651-0072 (JP)
(74) Representative: Manitz Finsterwald Patent- und Rechtsanwaltspartnerschaft mbB

(56) References cited:
- CN-A- 114 539 684
- JP-A- 2021 053 988

## Description

### BACKGROUND OF THE INVENTION

### Field of the Invention

The present invention relates to a medical rubber composition and a medical rubber product, and more specifically relates to a technique for improving non-elution properties of a medical rubber product.

### Background Art

Medical rubber products to be applied to pharmaceutical containers are required to have high quality properties and high physical properties. For example, the quality and properties that are required for a medical rubber plug for sealing or plugging an opening of a vial in which a formulation such as an antibiotic is stored should comply with Test for Rubber Closure for Aqueous Infusions in the 17th edition of the Japanese Pharmacopoeia in order to use the medical rubber plug. Furthermore, it is essential that the medical rubber plug for, for example, sealing an opening of a vial has many properties such as gas permeation resistance, non-elution properties, high cleanability, chemical resistance, needle penetration resistance, self-sealability, and high slidability.

For medical rubber products such as medical rubber plugs, halogenated isobutylene-isoprene-rubber is used because of its excellent gas (air, water vapor) permeation resistance. If heat is applied during kneading and molding of the halogenated isobutylene-isoprene-rubber, halogen gas is generated, so that equipment may be corroded. An acid acceptor has been known to be blended in the halogenated isobutylene-isoprene-rubber in order to accept an acid in the halogen gas.

For example, Japanese Laid-Open Patent Publication No. 2021-80370 discloses a medical rubber composition that contains (a) a base polymer containing halogenated isobutylene-isoprene-rubber and (b) a liquid polymer, and further contains hydrotalcite or magnesium oxide as an acid acceptor.

Japanese Laid-Open Patent Publication No. 2021-53988 discloses a slidable rubber material precursor carrying a silicone-rubber-overcoating coating layer, and the slidable rubber material precursor carrying the silicone-rubber-overcoating coating layer includes: a rubber product that contains at least one rubber component selected from isobutylene-isoprene-rubber, halogenated isobutylene-isoprene-rubber, and chloroprene rubber, a filler, a vulcanizing agent, and a vulcanization aid, and further contains an acid acceptor selected from magnesium oxide, zinc oxide, and natural or synthetic hydrotalcite; and an interfacial modification layer that covers the rubber product, and is obtained by containing an amino-modified silicone compound and/or reacting with surface molecules of the rubber product.

CN 114 539 684 A discloses the use of a halogenated butyl rubber material for an injection medicine, e.g. as a rubber stopper, wherein the rubber composition comprises calcium carbonate as an acidic substance adsorbent.

If a medical rubber product obtained by molding a medical rubber composition in which halogenated isobutylene-isoprene-rubber is used is stored in a state of being in contact with a pharmaceutical preparation (for example, saline), a pH of the pharmaceutical preparation (for example, saline) may become higher according to the storage period being longer.

The present invention has been made in view of the aforementioned circumstances, and an object of the present invention is to provide a medical rubber composition that has an acid acceptor blended in halogenated isobutylene-isoprene-rubber, and can allow a pH of a pharmaceutical preparation to be maintained while inhibiting corrosion of equipment due to halogen gas. Another object of the present invention is to provide a medical rubber product having excellent non-elution properties.

### SUMMARY OF THE INVENTION

A medical rubber composition of the present invention includes: a base polymer containing halogenated isobutylene-isoprene-rubber; and an acid acceptor, wherein the acid acceptor contains magnesium oxide or zinc oxide and an amount of the acid acceptor to be blended is 1 part by mass to 3 parts by mass with respect to 100 parts by mass of the base polymer, and a BET specific surface area of the acid acceptor is 150 m²/g to 200 m²/g, wherein the BET specific surface area of the acid acceptor is determined by the method defined in the description.

The inventors of the present invention investigated the reason why a medical rubber product obtained by molding a conventional medical rubber composition in which an acid acceptor was blended in halogenated isobutylene-isoprene-rubber caused increase of a pH of a pharmaceutical preparation according to the storage period being longer, and have found that the acid acceptor is eluted into the pharmaceutical preparation and affects the pH. The inventors of the present invention have found that, in a case where the acid acceptor contains magnesium oxide or zinc oxide, the BET specific surface area of the acid acceptor is specified as 150 m²/g to 200 m²/g, and an amount of the acid acceptor to be blended is set as 1 part by mass to 3 parts by mass with respect to 100 parts by mass of the base polymer, the obtained medical rubber composition can allow a pH of the pharmaceutical preparation to be maintained while inhibiting corrosion of equipment due to halogen gas, and have completed the present invention.

The present invention can provide the medical rubber composition that can allow a pH of the pharmaceutical preparation to be maintained while inhibiting corrosion of equipment due to halogen gas. Furthermore, the present invention can provide the medical rubber product having excellent non-elution properties.

### BRIEF DESCRIPTION OF THE DRAWINGS

FIG. 1 illustrates a medical rubber product (stopper) according to one embodiment of the present invention;
FIG. 2 illustrates a medical rubber product (rubber plug) according to one embodiment of the present invention;
FIG. 3 illustrates a medical rubber product (vial rubber plug) according to one embodiment of the present invention; and
FIG. 4 illustrates a medical rubber product (rubber plug for vacuum blood collecting tube) according to one embodiment of the present invention.

### DETAILED DESCRIPTION

### <Medical rubber composition>

A medical rubber composition of the present invention contains a base polymer containing halogenated isobutylene-isoprene-rubber, and an acid acceptor, and an amount of the acid acceptor to be blended is 1 part by mass to 3 parts by mass with respect to 100 parts by mass of the base polymer, and a BET specific surface area of the acid acceptor is 150 m²/g to 200 m²/g.

### (Base polymer)

Firstly, the base polymer that contains halogenated isobutylene-isoprene-rubber and is used in the present invention will be described. Examples of the halogenated isobutylene-isoprene-rubber include a chlorinated isobutylene-isoprene-rubber, a brominated isobutylene-isoprene-rubber, and a bromide of a copolymer of isobutylene and p-methylstyrene. As the halogenated isobutylene-isoprene-rubber, the chlorinated isobutylene-isoprene-rubber or the brominated isobutylene-isoprene-rubber is preferable. The chlorinated isobutylene-isoprene-rubber or the brominated isobutylene-isoprene-rubber is, for example, obtained by addition of or substitution with chlorine or bromine at an isoprene structure moiety in isobutylene-isoprene-rubber, specifically, at a double bond and/or a carbon atom adjacent to a double bond. The isobutylene-isoprene-rubber is a copolymer obtained by polymerizing isobutylene and a small amount of isoprene.

A halogen content in the halogenated isobutylene-isoprene-rubber is preferably 0.5 mass% or more, more preferably 1 mass% or more, and even more preferably 1.5 mass% or more, and preferably 5 mass% or less, more preferably 4 mass% or less, and even more preferably 3 mass% or less.

Specifically, the chlorinated isobutylene-isoprene-rubber is, for example, at least one of CHLOROBUTYL 1066 [stabilizer: NS, halogen content: 1.26%, Mooney viscosity: 38 ML₁₊₈ (125°C), specific gravity: 0.92] manufactured by JAPAN BUTYL Co., Ltd., and LANXESS X_BUTYL CB1240 manufactured by LANXESS.

Specifically, the brominated isobutylene-isoprene-rubber is, for example, at least one of BROMOBUTYL 2255 [stabilizer: NS, halogen content: 2.0%, Mooney viscosity: 46 ML₁₊₈ (125°C), specific gravity: 0.93] manufactured by JAPAN BUTYL Co., Ltd., and LANXESS X_BUTYL BBX2 and X_BUTYL BB 2030 manufactured by LANXESS.

In the present invention, as the halogenated isobutylene-isoprene-rubber, the chlorinated isobutylene-isoprene-rubber or the brominated isobutylene-isoprene-rubber is preferably used.

The base polymer may contain a rubber component other than the halogenated isobutylene-isoprene-rubber. Examples of the other rubber component include butyl-based rubber, isoprene rubber, butadiene rubber, styrene butadiene rubber, natural rubber, chloroprene rubber, nitrile-based rubber such as acrylonitrile butadiene rubber, hydrogenated nitrile rubber, norbornene rubber, ethylene-propylene rubber, ethylene-propylene-diene rubber, acrylic rubber, ethylene-acrylate rubber, fluororubber, chlorosulfonated polyethylene rubber, epichlorohydrin rubber, silicone rubber, urethane rubber, polysulfide rubber, phosphazene rubber, and 1,2-polybutadiene. One of them may be used alone, or two or more of them may be used in combination.

In a case where the other rubber component is used, a content of the halogenated isobutylene-isoprene-rubber in the base polymer is preferably 90 mass% or more, more preferably 95 mass% or more, and even more preferably 98 mass% or more. It is also preferable that the base polymer is merely formed of the halogenated isobutylene-isoprene-rubber.

### (Acid acceptor)

Next, the acid acceptor used in the present invention will be described. The acid acceptor is blended in order to absorb halogen gas (for example, hydrogen chloride gas or hydrogen bromide gas) generated during crosslinking of the halogenated isobutylene-isoprene-rubber. The acid acceptor is not particularly limited as long as the acid acceptor can be blended in the medical rubber composition, and examples of the acid acceptor include metal oxides, metal hydroxides, and hydrotalcite.

Specific examples of the metal oxide include magnesium oxide (MgO), zinc oxide (ZnO), calcium oxide (CaO), and aluminum oxide (Al₂O₃). Specific examples of the metal hydroxide include magnesium hydroxide (Mg(OH)₂), zinc hydroxide (Zn(OH)₂), calcium hydroxide (Ca(OH)₂), and aluminum hydroxide (Al(OH)₃). Specific examples of the hydrotalcite include Mg-Al-based hydrotalcites such as Mg_{4.5}Al₂(OH)₁₃CO₃·3.5H₂O, Mg_{4.5}Al₂(OH)₁₃CO₃, Mg₄Al₂(OH)₁₂CO₃·3.5H₂O, Mg₆Al₂(OH)₁₆CO₃·4H₂O, Mg₅Al₂(OH)₁₄CO₃·4H₂O, and Mg₃Al₂(OH)₁₀CO₃·1.7H₂O. Either of natural hydrotalcite or synthetic hydrotalcite may be used. One of these acid acceptors may be used alone, or two or more of them may be used in combination.

In the present invention, the acid acceptor is preferably the metal oxide and/or metal hydroxide, is more preferably at least one selected from the group consisting of MgO, ZnO, Ca(OH)₂, and Al(OH)₃. According to the present invention, the acid acceptor contains MgO or ZnO.

Specifically, the magnesium oxide used as the acid acceptor is, for example, a commercially available product such as "STARMAG R" manufactured by Konoshima Chemical Co., Ltd.

The BET specific surface area of the acid acceptor used in the present invention is preferably 150 m²/g or more, more preferably 160 m²/g or more, and even more preferably 170 m²/g or more, and preferably 200 m²/g or less, more preferably 190 m²/g or less, and even more preferably 180 m²/g or less. In a case where the BET specific surface area of the acid acceptor is within the above-described range, halogen gas generated during processing is appropriately absorbed by the acid acceptor. The BET specific surface area of the acid acceptor represents a value measured by a gas phase adsorption method in which nitrogen gas is used as adsorbed gas. For example, the BET specific surface area can be measured according to the measurement method specified in JIS Z 8830:2013 "Determination of the specific surface area of powders (solids) by gas adsorption-BET method".

The maximum particle diameter of the acid acceptor used in the present invention is preferably less than 20 µm, more preferably 18 µm or less, and even more preferably 15 µm or less. In a case where the maximum particle diameter of the acid acceptor is less than 20 µm, defects that the materials themselves become white spot(s) of foreign material can be reduced, and outer appearance of the product becomes better. The maximum particle diameter of the acid acceptor can be measured by, for example, a laser diffraction/scattering method (ultrasonication).

The average particle diameter of the acid acceptor used in the present invention is preferably 2.0 µm or more and more preferably 3.0 µm or more, and preferably 5.0 µm or less and more preferably 4.0 µm or less. In a case where the average particle diameter of the acid acceptor is within the above-described range, when the acid acceptor is dispersed in the base polymer, aggregation of the acid acceptor does not cause white spot defects, and the acid acceptor can be more uniformly dispersed. The average particle diameter of the acid acceptor can be measured by, for example, a laser diffraction/scattering method (ultrasonication). For this average particle diameter, the volume-based D₅₀ value is used as the average particle diameter (µm).

An amount of the acid acceptor to be blended in the medical rubber composition of the present invention is preferably 1.0 part by mass or more, more preferably 1.2 parts by mass or more, and even more preferably 1.5 parts by mass or more, with respect to 100 parts by mass of the base polymer, and preferably 3.0 parts by mass or less, more preferably 2.5 parts by mass or less, and even more preferably 2.0 parts by mass or less, with respect to 100 parts by mass of the base polymer. In a case where the content of the acid acceptor is 1.0 part by mass or more, the acid accepting effect with respect to halogen gas generated during the rubber kneading/molding processes is enhanced, and corrosion of equipment used in these processes due to halogen gas can be inhibited. In a case where the content of the acid acceptor is 3.0 parts by mass or less, the acid acceptor is inhibited from being eluted from the medical rubber product into a pharmaceutical preparation, and, furthermore, processability (appropriateness of Mooney viscosity) during molding also becomes better.

A molar ratio (halogen/acid acceptor) of halogen in the halogenated isobutylene-isoprene-rubber to the acid acceptor is preferably 1.0 or less, more preferably 0.8 or less, and even more preferably 0.7 or less, and preferably 0.3 or more, more preferably 0.4 or more, and even more preferably 0.5 or more. In a case where the molar ratio (halogen/acid acceptor) is 1.0 or less, the acid accepting effect with respect to halogen gas generated in the rubber kneading/molding processes is further enhanced, and corrosion of equipment used in these processes due to halogen gas can be further inhibited. In a case where the molar ratio (halogen/acid acceptor) is 0.3 or more, the acid acceptor is further inhibited from being eluted from the medical rubber product into a pharmaceutical preparation, and, furthermore, processability (appropriateness of Mooney viscosity) during molding also becomes better. The molar ratio (halogen/acid acceptor) can be calculated according to the following equation.

Molar ratio (halogen/acid acceptor)={ (parts by mass of halogenated isobutylene-isoprene-rubber×halogen content in halogenated isobutylene-isoprene-rubber)/halogen molecular weight}/{parts by mass of acid acceptor/acid acceptor molecular weight}

### (Vulcanizing agent)

Preferably, the medical rubber composition of the present invention further contains a vulcanizing agent. The vulcanizing agent is blended in order to vulcanize the halogenated isobutylene-isoprene-rubber component contained in the base polymer. The vulcanizing agent is not particularly limited as long as the halogenated isobutylene-isoprene-rubber can be vulcanized by the vulcanizing agent. Examples of the vulcanizing agent include sulfur, organic peroxides, and triazine derivatives. One of them may be used alone, or two or more of them may be used in combination.

Examples of the sulfur include powdery sulfur, sulfur fine powder, precipitated sulfur, colloidal sulfur, and sulfur chloride.

Examples of the organic peroxide include a dialkyl peroxide, a peroxy ester, a peroxy ketal, and a hydroperoxide. Examples of the dialkyl peroxide include di(2-t-butylperoxyisopropyl)benzene, dicumyl peroxide, 2,5-dimethyl-2,5-di(t-butylperoxy)hexane, t-butylcumyl peroxy, di-t-hexylperoxy, di-t-butylperoxy, and 2,5-dimethyl-2,5-di(t-butylperoxy)hexyne-3. Examples of the peroxy ester include t-butylperoxy maleate, t-butylperoxy-3,3,5-trimethyl cyclohexanoate, t-butylperoxy laurate, t-butylperoxyisopropyl monocarbonate, t-hexylperoxy benzoate, 2,5-dimethyl-2,5-di(benzoylperoxy)hexane, t-butylperoxy acetate, and t-butylperoxy benzoate. Examples of the peroxy ketal include 1,1-di(t-hexylperoxy)-3,3,5-trimethylcyclohexane, 1,1-di(t-hexylperoxy)cyclohexane, 1,1-di(t-butylperoxy)-2-methylcyclohexane, 1,1-di(t-butylperoxy)cyclohexane, 2,2-di(t-butylperoxy)butane, n-butyl-4,4-di(t-butylperoxy)valerate, and 2,2-di(4,4-di(t-butylperoxy)cyclohexyl)propane. Examples of the hydroperoxide include p-menthane hydroperoxide and diisopropylbenzene hydroperoxide.

Examples of the triazine derivative include compounds represented by general formula (1). 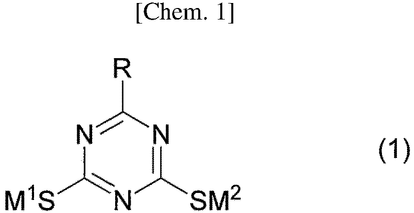 [in the formula, R represents -SH, -OR¹, -SR², -NHR³, or -NR⁴R⁵ (R¹, R², R³, R⁴, and R⁵ each represent an alkyl group, an alkenyl group, an aryl group, an aralkyl group, an alkylaryl group, or a cycloalkyl group. R⁴ and R⁵ may be the same or different from each other.). M¹ and M² each represent H, Na, Li, K, 1/2Mg, 1/2Ba, 1/2Ca, an aliphatic primary amine, secondary amine, or tertiary amine, a quaternary ammonium salt, or a phosphonium salt. M¹ and M² may be the same or different from each other.]

In the general formula (1), examples of the alkyl group include a C1 to C12 alkyl group such as a methyl group, an ethyl group, an n-propyl group, an isopropyl group, an n-butyl group, an isobutyl group, a tert-butyl group, an n-pentyl group, an isopentyl group, a tert-pentyl group, an n-hexyl group, a 1,1-dimethylpropyl group, an octyl group, an isooctyl group, a 2-ethylhexyl group, a decyl group, and a dodecyl group. Examples of the alkenyl group include a C1 to C12 alkenyl group such as a vinyl group, an allyl group, a 1-propenyl group, an isopropenyl group, a 2-butenyl group, a 1,3-butadienyl group, and a 2-pentenyl group. Examples of the aryl group include monocyclic or condensed polycyclic aromatic hydrocarbon groups, and include a C6 to C14 aryl group such as a phenyl group, a naphthyl group, an anthryl group, a phenanthryl group, and an acenaphthylenyl group. Examples of the aralkyl group include a C7 to C19 aralkyl group such as a benzyl group, a phenethyl group, a diphenylmethyl group, a 1-naphthylmethyl group, a 2-naphthylmethyl group, a 2,2-diphenylethyl group, a 3-phenylpropyl group, a 4-phenylbutyl group, a 5-phenylpentyl group, a 2-biphenylylmethyl group, a 3-biphenylylmethyl group, and a 4-biphenylylmethyl group. Examples of the alkylaryl group include a C7 to C19 alkylaryl group such as a tolyl group, a xylyl group, and an octylphenyl group. Examples of the cycloalkyl group include a C3 to C9 cycloalkyl group such as a cyclopropyl group, a cyclobutyl group, a cyclopentyl group, a cyclohexyl group, a cycloheptyl group, a cyclooctyl group, and a cyclononyl group.

Specific examples of the triazine derivative represented by the general formula (1) include 2,4,6-trimercapto-s-triazine, 2-methylamino-4,6-dimercapto-s-triazine, 2-(n-butylamino)-4,6-dimercapto-s-triazine, 2-octylamino-4,6-dimercapto-s-triazine, 2-propylamino-4,6-dimercapto-s-triazine, 2-diallylamino-4,6-dimercapto-s-triazine, 2-dimethylamino-4,6-dimercapto-s-triazine, 2-dibutylamino-4,6-dimercapto-s-triazine, 2-di(iso-butylamino)-4,6-dimercapto-s-triazine, 2-dipropylamino-4,6-dimercapto-s-triazine, 2-di(2-ethylhexyl)amino-4,6-dimercapto-s-triazine, 2-dioleylamino-4,6-dimercapto-s-triazine, 2-laurylamino-4,6-dimercapto-s-triazine, 2-anilino-4,6-dimercapto-s-triazine, and sodium salts or disodium salts thereof.

In the present invention, sulfur or the triazine derivative is preferably used as the vulcanizing agent.

A content of the vulcanizing agent in the medical rubber composition of the present invention is preferably 0.1 parts by mass or more, more preferably 0.2 parts by mass or more, and even more preferably 0.3 parts by mass or more, with respect to 100 parts by mass of the base polymer, and preferably 5.0 parts by mass or less, more preferably 3.0 parts by mass or less, and even more preferably 1.0 part by mass or less, with respect to 100 parts by mass of the base polymer. In a case where the content of the vulcanizing agent is within the above-described range, rubber having good rubber properties (hardness, tension, Cset) and good processability (little burning) can be obtained.

The medical rubber composition of the present invention preferably contains no vulcanization accelerator because a vulcanization accelerator may remain in a final rubber product and may be eluted into a pharmaceutical liquid in a syringe or vial. Examples of the vulcanization accelerator include guanidine-based accelerators (e.g. diphenylguanidine), thiuram-based accelerators (e.g. tetramethylthiuram disulfide, tetramethylthiuram monosulfide), dithiocarbamate-based accelerators (e.g. zinc dimethyldithiocarbamate), thiazole-based accelerators (e.g. 2-mercaptobenzothiazole, dibenzothiazyl disulfide), and sulfenamide-based accelerators (e.g. N-cyclohexyl-2-benzothiazole sulfenamide, N-t-butyl-2-benzothiazole sulfenamide).

### (Filler)

The medical rubber composition of the present invention may further contain a filler. Examples of the filler include an inorganic filler such as clay and talc, and resin powder of an olefin resin, styrene-based elastomer, or ultrahigh molecular weight polyethylene (UHMWPE). Among them, as the filler, the inorganic filler is preferable, and clay or talc is more preferable. The filler functions to adjust rubber hardness of the medical rubber product, and also functions as an extender to reduce production cost of the medical rubber product.

Examples of the clay include burned clay and kaolin clay. Specific examples of the clay include SILLITIN (registered trademark) Z manufactured by Hoffmann Mineral, SATINTONE (registered trademark) W manufactured by Engelhard Corporation, NN Kaolin Clay manufactured by Tsuchiya Kaolin Kogyo, and PoleStar 200R manufactured by Imerys Specialities Japan.

Specific examples of the talc include High toron A manufactured by Takehara Kagaku Kogyo Co., Ltd., MICRO ACE (registered trademark) K-1 manufactured by NIPPON TALC CO., LTD., and ImerFlex (registered trademark) T20 manufactured by Imerys Specialities Japan.

A content of the filler in the medical rubber composition of the present invention is preferably set as appropriate according to, for example, the rubber hardness of a target medical rubber product. The content of the filler in the medical rubber composition of the present invention is, for example, preferably 5 parts by mass or more, more preferably 10 parts by mass or more, and even more preferably 20 parts by mass or more, with respect to 100 parts by mass of the base polymer, and preferably 200 parts by mass or less, more preferably 150 parts by mass or less, and even more preferably 100 parts by mass or less, with respect to 100 parts by mass of the base polymer.

### (Other components)

Furthermore, in the medical rubber composition of the present invention, in addition to the above-described components, a coloring agent such as titanium oxide and carbon black, a lubricant such as stearic acid and low density polyethylene (LDPE), a processing aid, polyethylene glycol acting as a crosslinking activator, or the like may be blended at an appropriate proportion.

The medical rubber composition of the present invention is obtained by kneading the base polymer, the acid acceptor, and other blending materials to be added as necessary. For example, the kneading can be performed by using an open roll, a sealed kneader, or the like. The kneaded product is preferably formed into a ribbon-like shape, a sheet-like shape, a pellet-like shape, or the like, and is more preferably formed into a sheet-like shape.

A Mooney viscosity (ML₁₊₄ (100°C)) of the medical rubber composition of the present invention is preferably 60 or more and more preferably 62 or more, and is preferably 75 or less and more preferably 70 or less. In a case where the Mooney viscosity (ML₁₊₄ (100°C)) is within the above-described range, kneadability of the medical rubber composition becomes better, and processability is further enhanced.

### <Medical rubber product>

The present invention includes a medical rubber product formed by molding the medical rubber composition of the present invention. The medical rubber product of the present invention can be produced by a conventional production method. For example, the medical rubber composition formed into a ribbon-like shape, a sheet-like shape, a pellet-like shape, or the like is press-molded, thereby obtaining a medical rubber product having a desired shape. A crosslinking reaction (vulcanization reaction) of the medical rubber composition progresses during the pressing process. The molding temperature is, for example, preferably 130°C or higher and more preferably 140°C or higher, and preferably 200°C or lower and more preferably 190°C or lower. The molding time is preferably two minutes or longer and more preferably three minutes or longer, and preferably 60 minutes or shorter and more preferably 30 minutes or shorter. The molding pressure is preferably 0.1 MPa or more and more preferably 0.2 MPa or more, and preferably 10 MPa or less and more preferably 8 MPa or less.

An unnecessary portion of a molded product obtained after the press-molding is cut and removed, and the molded product can have a predetermined shape. The obtained molded product is washed, sterilized, dried, and packed, thereby producing a medical rubber product.

Furthermore, a resin film may be integrally laminated on the medical rubber product of the present invention as in conventional art. Examples of the resin film include an inactive resin film formed of, for example, polytetrafluoroethylene (PTFE), tetrafluoroethylene-ethylene copolymer (ETFE), a modified product thereof, or ultra-high density polyethylene (UHDPE).

The resin film is, for example, press-molded in a state of being stacked on the sheet-shaped rubber composition, and may thus be integrated with the medical rubber product formed after the press-molding.

The medical rubber product of the present invention can be suitably used as a medical rubber product that can particularly come into contact with a pharmaceutical preparation, blood, or the like. Specific examples of the medical rubber product of the present invention include medical rubber plugs such as rubber plugs of containers for various pharmaceutical preparations such as a liquid preparation, a powder preparation, and a freeze-dried preparation, and rubber plugs for vacuum blood collecting tubes, and sliding or sealing components such as a syringe stopper.

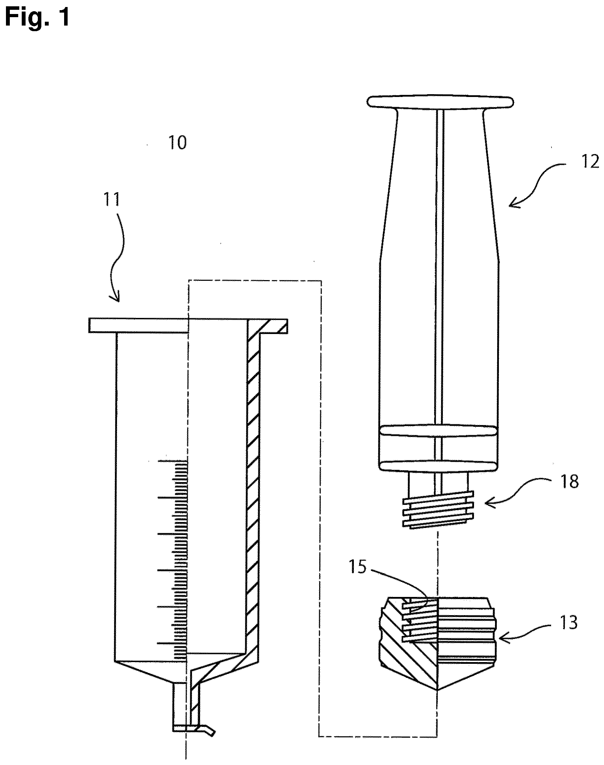

FIG. 1 is an exploded view of a medical syringe, that is, a syringe called a prefillable syringe, for which the medical rubber product of the present invention is used. The half of each of a syringe 11 and a stopper 13 is shown as a cross-sectional view in FIG. 1. A prefillable syringe 10 includes the syringe 11 having a cylindrical shape, a plunger 12 that is combined with the syringe 11 and can reciprocate in the syringe 11, and the stopper 13 attached to the head of the plunger 12. The stopper 13 is molded from the medical rubber composition of the present invention. A laminate film may be stacked on the surface of the stopper 13 in order to improve slidability.

The plunger 12 is, for example, formed of a resin plate having a cross-shaped transverse cross-section, and has, at the head of the plunger 12, a head portion 18 to which the stopper 13 is attached. The head portion 18 is formed of resin and integrated with the plunger 12, and is processed into a male screw shape. The stopper 13 has a substantially cylindrical shape having a short axis, and the end surface has, for example, a mountain-like shape forming an obtuse angle and having a protruding axial center portion. The stopper 13 has a female-screw-shaped fitting recess 15 that is engraved from the rear end surface in the axial direction. By screwing the head portion 18 of the plunger 12 into the fitting recess 15 of the stopper 13, the stopper 13 is attached to the head of the plunger 12.

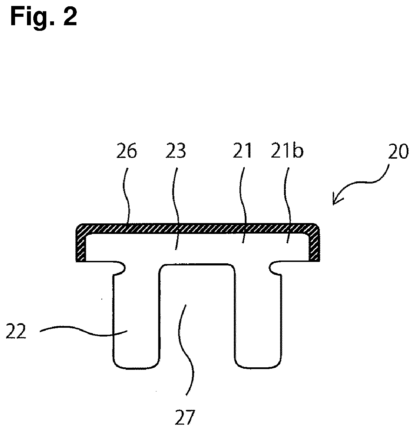

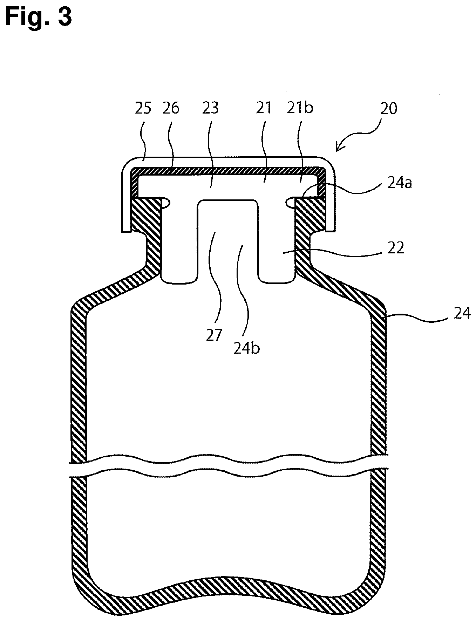

FIG. 2 is a schematic cross-sectional view of one specific example of a rubber plug 20 as the medical rubber product of the present invention. FIG. 3 illustrates a state where an opening 24b of a container 24 filled with a pharmaceutical preparation is sealed by the rubber plug 20.

The rubber plug 20 has a top plate 21 and a plug leg 22. The top plate 21 has a puncture portion 23 that can be punctured with an injector needle of an injector, and a flange portion 21b that comes into contact with an upper edge surface 24a of a container opening of the medical container 24. The plug leg 22 protrudes from the lower surface of the top plate 21, and is inserted into the medical container opening. The plug leg 22 has a substantially cylindrical shape, and has a cut portion 27. A nylon film layer 26 is disposed on the top surface portion of the top plate 21 of the rubber plug 20. In a case where the nylon film layer 26 is disposed on the top surface portion of the rubber plug 20, mechanical transportability can be ensured while a pharmaceutical preparation is produced. In a case where the nylon film layer 26 is disposed on the top surface portion of the rubber plug 20, surface lubricity of the top surface portion can be enhanced, and needle puncture fragments can be prevented from being generated during puncturing with an injector needle.

In the state illustrated in FIG. 3, a vial for storing a freeze-dried preparation is used as the container 24. In a case where the liquid pharmaceutical preparation stored in the container 24 is a pharmaceutical liquid for injection, the puncture portion 23 of the top plate 21 is punctured with an injector needle of an injector, and the pharmaceutical liquid for injection is suctioned into the injector without opening the rubber plug 20. The rubber plug 20 is not opened in order to prevent a foreign material from being mixed in the pharmaceutical liquid for injection in the container 24.

A metal or resin cap 25 that can cover the opening 24b of the container 24 and the rubber plug 20 is disposed on the top plate 21. The rubber plug 20 together with the opening 24b is sealed with the cap 25 in order to prevent bacteria from being adhered to the puncture portion 23 at a portion that is punctured by an injector needle and mixed into the pharmaceutical liquid for injection through the injector needle. As the type of the cap 25, for example, a flip-off cap, a pull-top cap, or a clean cap can be used. In a case where a large amount of pharmaceutical liquid for injection is used as in hospitals, a clean cap that can be unsealed with one hand and easily operated is suitably used.

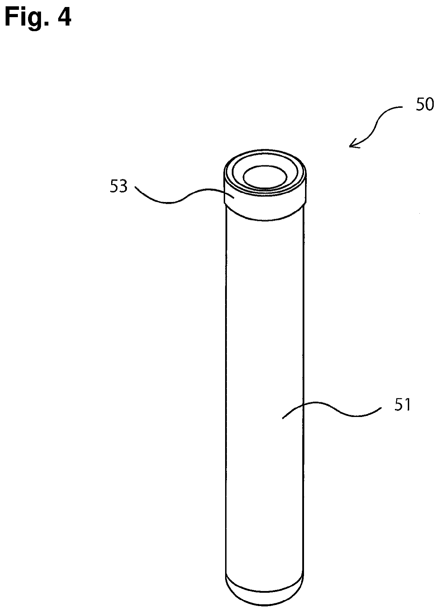

FIG. 4 illustrates one example of a vacuum blood collecting tube. A vacuum blood collecting tube 50 includes a bottomed tube 51, and a rubber plug 53 for seating an opening of the bottomed tube 51. This rubber plug 53 is formed of the medical rubber composition of the present invention. The vacuum blood collecting tube 50 is designed so as to automatically collect blood by reducing pressure in the blood collecting tube.

### [Examples]

Hereinafter, the present invention will be described in detail by means of examples, but the present invention is not limited to the following examples, and any of modifications and implementation modes which do not depart from the gist of the present invention is included in the scope of the present invention.

### [Preparation of medical rubber composition]

The materials indicated in Table 1 were kneaded to prepare medical rubber compositions. The materials were kneaded at 80°C to 140°C for 5 to 30 minutes by using a sealed-type pressure kneader.

**[Table 1]**

| Medical rubber composition No. | | | 1 | 2 | 3 | 4 | 5 | 6 | 7 | 8 | 9 | 10 | 11 | 12 |
|---|---|---|---|---|---|---|---|---|---|---|---|---|---|---|
| Blending (parts by mass) | Halogenated isobutylene-isoprene-rubber | | 100 | 100 | 100 | 100 | 100 | 100 | 100 | 100 | 100 | 100 | 100 | 100 |
| | Acid acceptor | Acid acceptor 1 (BET specific surface area of 178 m²/g) | 12.0 | 9.0 | 6.0 | 5.0 | - | - | 3.0 | 2.5 | 2.0 | 1.5 | 1.0 | 0.5 |
| | | Acid acceptor 2 (BET specific surface area of 65 m²/g) | - | - | - | - | 1.5 | - | - | - | - | - | - | - |
| | | Acid acceptor 3 (BET specific surface area of 47 m²/g) | - | - | - | - | - | 1.5 | - | - | - | - | - | - |
| | Sulfur | | 0.3 | 0.3 | 0.3 | 0.3 | 0.3 | 0.3 | 0.3 | 0.3 | 0.3 | 0.3 | 0.3 | 0.3 |
| | Talc | | 80 | 80 | 80 | 80 | 80 | 80 | 80 | 80 | 80 | 80 | 80 | 80 |
| | Titanium oxide | | 3.0 | 3.0 | 3.0 | 3.0 | 3.0 | 3.0 | 3.0 | 3.0 | 3.0 | 3.0 | 3.0 | 3.0 |
| | Carbon black | | 0.3 | 0.3 | 0.3 | 0.3 | 0.3 | 0.3 | 0.3 | 0.3 | 0.3 | 0.3 | 0.3 | 0.3 |
| Evaluation | Mooney viscosity (ML₁₊₄ (100°C)) | | 83 | 78 | 70 | 69 | 62 | 62 | 66 | 64 | 63 | 62 | 61 | 60 |
| | Outer appearance inspection | | E | E | E | E | G | P | E | E | E | E | E | E |
| | Eluted material test | Maximum pH difference between test liquid and blank test liquid | 1.29 | 1.20 | 1.19 | 1.09 | 2.70 | 3.70 | 0.98 | 1.01 | 0.99 | 0.98 | 0.96 | 0.95 |
| | | Determination | P | P | P | P | P | P | E | E | E | E | E | E |
| | Corrosion of equipment | | E | E | E | E | E | E | E | E | E | G | G | P |

The details of the used blending materials are as follows.
Brominated isobutylene-isoprene-rubber: BROMOBUTYL 2255 manufactured by JAPAN BUTYL Co., Ltd., [bromine content: 2.0%]
Acid acceptor 1: STARMAG (registered trademark) R manufactured by Konoshima Chemical Co., Ltd. (magnesium oxide, BET specific surface area of 178 m²/g, maximum particle diameter of 13 µm, average particle diameter of 3.5 µm)
Acid acceptor 2: MAGSARAT (registered trademark) 150ST manufactured by Kyowa Chemical Industry Co., Ltd. (magnesium oxide, BET specific surface area of 65 m²/g, maximum particle diameter of 20 µm, average particle diameter of 4.8 µm)
Acid acceptor 3: STARMAG (registered trademark) M manufactured by Konoshima Chemical Co., Ltd. (magnesium oxide, BET specific surface area of 47 m²/g, maximum particle diameter of 262 µm, average particle diameter of 3.5 µm)
Sulfur: sulfur containing 5% of oil, manufactured by Tsurumi Chemical Industry Co., Ltd.
Talc: ImerFlex (registered trademark) T20 manufactured by Imerys Specialities
Titanium oxide: TIPAQUE (registered trademark) A-100 manufactured by ISHIHARA SANGYO KAISHA, LTD.
Carbon black: Thermax MT manufactured by Cancarb Limited

### [Evaluation method]

### (1) Mooney viscosity

A Mooney viscosity of the medical rubber composition was measured by a measurement method specified in JIS K6300-1:2013 "Determination of Mooney viscosity and pre-vulcanization characteristics with Mooney viscometer".

### (2) BET specific surface area (m²/g) of acid acceptor

The BET specific surface area was measured under the following conditions.
Measurement device: Macsorb HM model (manufactured by Mountech Co., Ltd.)
Pretreatment condition: 160°C, one hour
Method for measuring an amount of adsorbed gas: carrier gas method
Method for analyzing adsorption data: single point method

### (3) Particle diameter (µm) of acid acceptor

In a 100 mL beaker, 50 mL of ethanol was taken, and about 0.2 g of sample powder was put therein, and the obtained product was subjected to ultrasonication (UD-201 manufactured by TOMY SEIKO CO., LTD) for three minutes to prepare a dispersion. The prepared solution was measured by using a laser diffraction method by a particle size distribution analyzer (Microtrac HRA Model 9320-X100 manufactured by NIKKISO CO., LTD.). The volume-based D₅₀ value and the maximum particle diameter were obtained from the obtained particle size distribution.

### (4) Outer appearance inspection

The prepared medical rubber composition was press-molded at 15 to 20 atmospheres, at 165°C to 190°C, for 7 to 20 minutes, to produce a slab (length of 225 mm, width of 60 mm, thickness of 2 mm). The surface of the slab was visually observed, whether or not white spot(s) of foreign material caused due to the acid acceptor was found was determined, and the outer appearance was evaluated according to the following criteria.

Excellent (E): No white spot of foreign material caused due to the acid acceptor was found.

Good (G): Only one white spot of foreign material caused due to the acid acceptor was found.

Poor (P): Two or more white spots of foreign material caused due to the acid acceptor were found.

### (5) Eluted material test

The prepared medical rubber composition was press-molded at 15 to 20 atmospheres, at 165°C to 190°C, for 7 to 20 minutes, to produce a measurement sample (having φ17 mm and a thickness of 2 mm) for an eluted material test. Water-washing in which the produced measurement sample was washed with water or an alkali aqueous solution such as a sodium carbonate aqueous solution at 121°C for 30 minutes by using an autoclave, and was thereafter immersed in water, was performed three times. In a glass bottle, 200 g of saline was put, and a test liquid in which the washed sample was immersed was stored in a 40°C oven for predetermined time periods (0 h, 6 h, 24 h, 48 h, 72 h, 96 h, 168 h, 336 h, 504 h). A glass bottle merely containing 200 g of saline as a blank test liquid was also stored in a 40°C oven for the predetermined time periods. After elapse of the predetermined time periods, the test liquid and the blank test liquid were taken out from the ovens, and cooled to room temperature, a pH of each liquid was thereafter measured by a pH measurement machine (LAQUA D-210P type manufactured by HORIBA, Ltd.), and a difference in pH between the test liquid and the blank test liquid was calculated. The non-elution properties of the acid acceptor were evaluated according to the following criteria by using the maximum value (peak value) of the pH difference between the test liquid and the blank test liquid in the predetermined time periods. The less the pH difference was, the smaller the elution of the acid acceptor was.

Excellent (E): The maximum pH difference between the test liquid and the blank test liquid was less than 1.05.

Poor (P): The maximum pH difference between the test liquid and the blank test liquid was 1.05 or more.

### (6) Corrosion of equipment

The medical rubber composition was kneaded at 80°C to 140°C for 5 to 30 minutes. Thereafter, the metal surface of the kneader was visually checked, and corrosion of the equipment was evaluated according to the following criteria.

Excellent (E): Metal was not corroded, and the luster of the surface was maintained.

Good (G): Metal was slightly corroded, and the luster of the surface was reduced.

Poor (P): Metal was corroded, the luster of the surface was lost, and dirt was adhered and the color changed to brown if the equipment was continuously used.

Table 1 indicates the evaluation result of each item.

As is apparent from the results in Table 1, the medical rubber composition of the present invention which contained the base polymer containing the halogenated isobutylene-isoprene-rubber, and the acid acceptor, and in which the content of the acid acceptor was 1 part by mass to 3 parts by mass with respect to 100 parts by mass of the base polymer, and the BET specific surface area of the acid acceptor was 150 m²/g to 200 m²/g, was able to inhibit the acid acceptor from being eluted into a pharmaceutical preparation (was able to maintain a pH of the pharmaceutical preparation) while inhibiting corrosion of equipment due to halogen gas.

The present invention can be suitably applied to a medical rubber composition and a medical rubber product.

## Claims

1. A medical rubber composition comprising:
a base polymer containing halogenated isobutylene-isoprene-rubber; and
an acid acceptor, wherein
the acid acceptor contains magnesium oxide or zinc oxide,
wherein an amount of the acid acceptor to be blended is 1 part by mass to 3 parts by mass with respect to 100 parts by mass of the base polymer, and a BET specific surface area of the acid acceptor is 150 m²/g to 200 m²/g, wherein the BET specific surface area of the acid acceptor is determined by the method defined in the description.

2. The medical rubber composition according to claim 1, wherein the halogenated isobutylene-isoprene-rubber is chlorinated isobutylene-isoprene-rubber or brominated isobutylene-isoprene-rubber.

3. The medical rubber composition according to any one of claim 1 or 2, wherein a maximum particle diameter of the acid acceptor is less than 20 µm,

4. The medical rubber composition according to any one of claims 1 to 3, further comprising a vulcanizing agent.

5. The medical rubber composition according to any one of claims 1 to 4, further comprising a filler.

6. The medical rubber composition according to any one of claim 1 to 5, wherein a Mooney viscosity (ML₁₊₄ (100°C)) is 60 to 75, wherein the Mooney viscosity is determined by the method defined in the description.

7. A medical rubber product obtained by molding the medical rubber composition according to any one of claims 1 to 6.

8. The medical rubber product according to claim 7, wherein the medical rubber product is a stopper (13) for a syringe.

## Patentansprüche

1. Medizinische Kautschukzusammensetzung, umfassend:
ein Basispolymer, das halogenierten Isobutylen-Isopren-Kautschuk enthält; und
einen Säureakzeptor, wobei der Säureakzeptor Magnesiumoxid oder Zinkoxid enthält,
wobei eine Menge des zu mischenden Säureakzeptors 1 Massenteil bis 3 Massenteile, bezogen auf 100 Massenteile des Basispolymers, beträgt und eine spezifische BET-Oberfläche des Säureakzeptors 150 m²/g bis 200 m²/g beträgt, wobei die spezifische BET-Oberfläche des Säureakzeptors durch das in der Beschreibung definierte Verfahren bestimmt wird.

2. Medizinische Kautschukzusammensetzung nach Anspruch 1, wobei der halogenierte Isobutylen-Isopren-Kautschuk chlorierter Isobutylen-Isopren-Kautschuk oder bromierter Isobutylen-Isopren-Kautschuk ist.

3. Medizinische Kautschukzusammensetzung nach einem der Ansprüche 1 oder 2, wobei ein maximaler Teilchendurchmesser des Säureakzeptors weniger als 20 µm beträgt.

4. Medizinische Kautschukzusammensetzung nach einem der Ansprüche 1 bis 3, ferner umfassend ein Vulkanisationsmittel.

5. Medizinische Kautschukzusammensetzung nach einem der Ansprüche 1 bis 4, ferner umfassend einen Füllstoff.

6. Medizinische Kautschukzusammensetzung nach einem der Ansprüche 1 bis 5,
wobei eine Mooney-Viskosität (ML₁₊₄ (100°C)) 60 bis 75 beträgt, wobei die Mooney-Viskosität durch das in der Beschreibung definierte Verfahren bestimmt wird.

7. Medizinisches Kautschukprodukt, das durch Formen der medizinischen Kautschukzusammensetzung nach einem der Ansprüche 1 bis 6 erhalten wird.

8. Medizinisches Kautschukprodukt nach Anspruch 7, wobei das medizinische Kautschukprodukt ein Stopfen (13) für eine Spritze ist.

## Revendications

1. Composition de caoutchouc médical comprenant :
un polymère de base contenant un caoutchouc d'isobutylène-isoprène halogéné ; et
un accepteur acide, dans laquelle
l'accepteur acide contient de l'oxyde de magnésium ou de l'oxyde de zinc, dans laquelle une quantité de l'accepteur acide à mélanger est 1 partie en masse à 3 parties en masse par rapport à 100 parties en masse du polymère de base et une surface spécifique BET de l'accepteur acide est de 150 m²/g à 200 m²/g, la surface spécifique BET de l'accepteur acide étant déterminée par le procédé défini dans la description.

2. Composition de caoutchouc médical selon la revendication 1, dans laquelle le caoutchouc d'isobutylène-isoprène halogéné est un caoutchouc d'isobutylène-isoprène chloré ou un caoutchouc d'isobutylène-isoprène bromé.

3. Composition de caoutchouc médical selon l'une quelconque des revendications 1 ou 2, dans laquelle un diamètre de particule maximum de l'accepteur acide est de moins de 20 □m.

4. Composition de caoutchouc médical selon l'une quelconque des revendications 1 à 3, comprenant en outre un agent vulcanisant.

5. Composition de caoutchouc médical selon l'une quelconque des revendications 1 à 4, comprenant en outre une charge.

6. Composition de caoutchouc médical selon l'une quelconque des revendications 1 à 5, dans laquelle une viscosité de Mooney (ML₁₊₄ (100 °C)) est de 60 à 75, la viscosité de Mooney étant déterminée par le procédé défini dans la description.

7. Produit de caoutchouc médical obtenu en moulant la composition de caoutchouc médical selon l'une quelconque des revendications 1 à 6.

8. Produit de caoutchouc médical selon la revendication 7, dans lequel le produit de caoutchouc médical est un interrupteur (13) pour une seringue.
